# EUROPEAN PATENT APPLICATION

(11) **EP 0 552 559 A2**
(43) Date of publication of application: **28.07.1993**
(21) Application number: 92311630.5
(22) Date of filing: 21.12.1992
(51) Int. Cl.: C12N 15/62, C12N 15/12, C12N 15/40, C12N 9/50, C12N 15/82, C12N 5/10, A01H 5/00, A01N 63/00

(54) **Transgenic plants resistant to microbian infection**

(30) Priority: 23.12.1991 GB 9127250
(71) Applicant: UNILEVER PLC, London EC4P 4BQ (GB); UNILEVER N.V., NL-3013 AL Rotterdam (NL)
(72) Inventor: Scheffler, Brian, Cambridge Laboratory, Norwich, Norfolk, NR4 7UJ (GB); Bevan, Mike, Cambridge Laboratory, Norwich, Norfolk, NR4 7UJ (GB)
(74) Representative: Butler, David John

(57) **Abstract**

A method of enhancing the resistance of a plant, especially potato, to bacterial infection, comprising the step of genetically transforming the plant by means of a expression vector containing DNA encoding a peptide comprising a protease recognition site linked directly to the N-terminus of a protein having anti-bacterial activity, such as a magainin. Cleavage of the expressed protein product by a protease releases the anti-bacterial protein with its correct N-terminal sequence.

## Description

This invention relates to techniques for genetic modification of organisms, especially plants, to cause the organisms to express foreign proteins in active form.

Much research is now being conducted to modify the properties of plant cells by causing the expression in such cells of foreign proteins, often derived from organisms other than plants. One objective of immense potential benefit is the enhancement of the resistance of plants to diseases caused for example, by bacteria. Such objectives are often frustrated by the failure of the modified plant cell to express the foreign protein in a form that is sufficiently biologically active to provide the desired benefit. A common reason for this failure is that although the bulk of the protein sequence may be expressed almost correctly, it is found that one or more of the amino acids at the N-terminus of the expressed protein sequence are different from those found in the protein in its original source. In particular, the primary expression protein of a gene always has an N-terminal methionine residue.

It is an objective of the present invention to achieve greater control over the N-terminal sequence of expressed protein.

The invention provides an expression cassette comprising DNA encoding a peptide comprising a protease recognition site linked directly to the N-terminus of a desired protein. Preferably, the desired protein has anti-microbial activity, especially anti-bacterial activity. Examples are magainins or analogues thereof, or peptidyl-glycine-leucine carboxyamide (PGLa).

The invention includes an expression vector capable of genetically transforming a host organism, such as a plant, for example a Ti plasmid, comprising an expression cassette as defined in the foregoing paragraph.

The invention also includes a plant cell or a higher plant expressing in biologically active form a magainin or an analogue thereof, or peptidyl-glycine-leucine carboxyamide (PGLa).

An important embodiment of the invention is potato, in the form of whole plants, tubers or a cell culture, having enhanced resistance to bacterial infection conferred by expression therein of a magainin or an analogue thereof, or PGLa.

The invention also provides-a method of enhancing the resistance of an organism, such as plant to bacterial infection, comprising the step of genetically transforming the organism by means of a expression vector containing DNA encoding a peptide comprising a protease recognition site linked directly to the N-terminus of a protein having anti-bacterial activity.

If desired, the expression cassette can also encode a protease capable of acting on the recognition site. Alternatively, an appropriate protease can be supplied, for example, by separate expression in the organism, or applied exogenously, or use can be made of a protease that occurs naturally in the organism. If desired, expression of the protease can be triggered by an external stimulus to promote release of the desired protein in active form at a particular stage in the life cycle of the organism.

Expression or addition of the protease can therefore occur at a critical time, after an effective quantity of the recognition site/desired protein complex has been built up.

Initiation of production of the protease recognition site/desired protein complex, and/or of the protease, can be induced, for example, by exploiting natural defense against wounding or infection. Recognition sites from pathogenisis-related proteins can be used, for example promoters that respond to the natural production of salicylic acid by plants.

In this context, the "cassette" containing DNA encoding the protease recognition site and desired protein will usually be incorporated in an expression vector (plasmid) appropriate for the host organism. For plants, one example of a suitable expression vector is the Ti plasmid.

The invention will be particularly described in relation to the use of sequences from the tobacco etch virus (TEV) as a source of protease and recognition site sequences. Examples of desired proteins are bactericidal proteins, such as magainins.

Diseases caused by micro-organisms such as fungi, bacteria and viruses, as well as infestation by pests such as insects and nematodes, result in serious crop losses throughout the world. These diseases are generally combated by the breeding of plant varieties with enhanced degrees of natural inherited resistance to disease, and by the application of synthetic (or microbially-produced) compounds to combat the disease-causing micro-organism or its vector. There is a widely recognised need for more natural and environmentally-acceptable compounds and treatments to protect plants from pests and micro-organisms. In particular, it would be useful if plants could be made to produce anti-microbial or pesticidal compounds themselves, thus avoiding the need for exogenous application.

The skin of Xenopus laevis (South African clawed frog) contains several peptides that exhibit a wide spectrum of antimicrobial activity and their level of activity is comparable to other antibiotics (Bevins and Zasloff, 1990). These peptides are specifically located within the secretory vesicles of the granular glan (Giovannini et al, 1987; Moreira et al, 1988). Upon an outside stimulus (injection of adrenaline, stress induced by a predator or damage to the skin) these and other peptides are secreted (Giovannini et al, 1987; Bevins and Zasloff, 1990). Two peptides of major interest are magainin and peptidyl-glycine-leucine carboxyamide (PGLa). In addition to their antimicrobial activity, both of these peptides are similar in structure in that they are very basic and in certain conditions form amphipathic alpha helices. In addition, magainin and PGLa are not normally produced in active form; instead they are expressed as polyproteins and a proteolytic activity releases the active peptide.

The active form of magainin is 23 amino acids long and there are two natural types, Magainin 1 and Magainin 2. Most experiments conducted to understand the nature of magainin have used Magainin 2, because it is expressed at a higher level within the frog skin and it is a stronger antimicrobial agent. Magainin is active against several types of Gram-positive and Gram-negative bacteria, fungi and protozoa (Zasloff, 1987, Zasloff et al, 1988). In bacteria magainin has lytic activity and in protozoa it clearly disrupts the osmotic balance, causing the protozoa to expand and then burst (Zasloff, 1987; Zasloff et al, 1988). The antimicrobial activity of magainin appears to be that it can interact with negatively charged membranes and render them leaky. The leakage appears as membrane depolarization, which can be measured by its disruption of membrane-linked free-energy transduction (Juretic et al, 1989; Westerhoff et al, 1989a, 1989b; Williams et al, 1990). Although magainin can form an amphipathic alpha helical structure and is in theory long enough to span a bilipid membrane to form a transmembrane channel to account for this leakage, Raman spectroscopy studies have shown that it does not span the membrane (Williams et al, 1990).

Several analogs of magainin have been synthetically produced. Those analogs that have enhanced amphipathic alpha helical structure are more potent antimicrobial agents (Chen et al, 1988). Although these analogs can have up to 40-fold greater antimicrobial activity, they are typically only 20% more effective in decreasing membrane potential. It appears that the increase in the amphipathic alpha helical structure of these analogs helps protect them from proteolytic activity, and, therefore, they are active for a greater amount of time. When magainin peptides are degraded the membrane potential returns to normal, indicating that some resistant microbes may have strong proteolytic activity against magainin.

The active form of PGLa is 21 amino acids long. Although it has not been studied as extensively as magainin, it appears to have similar properties to magainin both in structure and activity (Soravia et al, 1988; Westerhoff et al, 1989a, 1989b; Bevins and Zasloff, 1990; Williams et al, 1990). The most important aspect of PGLa is that it acts synergistically with magainin, which increasing the overall antimicrobial activity by 20-50 fold (Bevins and Zasloff, 1990; Williams et al, 1990).

Although the effects of magainin and PGLa on membrane potential have been extensively studied, little is published on their overall toxicity to eukaryotes. Obviously, these peptides are not toxic to the frog nor do frogs appear to be toxic to human beings. In addition, the peptides have little or no hemolytic activity (Soravia et al, 1988).

The present invention involves, in one aspect, the use of a magainin (this term being used herein to include natural variants and synthetic analogues) as an antimicrobial and pesticidal agent against plant pathogens by genetically engineering a plant to produce the magainin.

Another important aspect of the invention is the construction and use of an expression cassette containing appropriate expression signals as well as the tobacco etch virus (TEV) protease gene together with a DNA sequence comprising part of the TEV leader sequence and a magainin gene fused to it, to produce an active form of magainin when the cassette is transferred-into and expressed in a plant.

The characteristics of all living organisms are governed by genes which are made from DNA. In principle, it is possible to transfer DNA-containing genes between any one living organism and any other so as to temporarily or permanently modify the characteristics of the second organism and its descendants with determined characteristics of the first organism. It is also possible to modify the genes within the DNA, or to create genes synthetically, which may give rise to modified or novel characteristics in the organism to which they are transferred.

To modify a plant it is necessary to construct a vector. The vector contains a DNA sequence termed the "expression cassette" which comprises (in part) the gene(s) to be transferred coupled with regulatory DNA sequences which ensure that the gene(s) is (are) properly expressed after transfer into the plant. Such regulatory DNA sequences determine the temporal and spatial (i.e. where in the plant) production of the protein product(s) encoded by the gene(s). Other parts of the expression cassette are DNA sequences and genes which ensure that the protein product encoded by a gene is translocated to the desired compartment of the plant cell, or to the extracellular space or into another cell and the the protein product which is the primary gene product is processed to the desired form.

A particularly useful context for the invention is the prevention or reduction of infection in potatoes by Erwinia, which causes "backleg" and "soft rot".

By way of example only, detailed steps by means of which appropriate expression vectors can be prepared are given later in this specification. The manipulation of DNA material in a suitably equipped laboratory is now a well-developed art, and the procedures required are well within the skill of those versed in this art. Many appropriate genomic and cDNA libraries, plasmids, restriction enzymes, and the various reagents and media which are required in order to perform such manipulations, are available commercially from suppliers of laboratory materials. For example, genomic and cDNA libraries can be purchased from Clontech Laboratories Inc. The steps given by way of example below are purely for the guidance of the reader of this specification, and the invention is in no way critically dependant upon the availability of one or more special starting materials. In practice, the skilled person has a wide range of materials from which to choose, and can exploit and adapt the published technology using acquired experience and materials that are most readily available in the scientific environment. For example, many plasmids fall into this category, having been so widely used and circulated within the relevant scientific community that they can now be regarded as common-place materials.

By way of example only, the production of transgenic magainin-expressing potato plants will now be described with reference to the accompanying drawings, which show:
- Figure 1:: Route for the construction of a TEV-promotor/magainin expression cassette.
- Figure 2:: Relevant sequence of vector PBI220.
- Figure 3:: Route for the construction of a TEV-promotor/PGLa expression cassette.
- Figure 4:: Electrophoresis gel showing in-vitro translation products.
- Figure 5:: Graphic results of "blackleg" resistance tests in potato plants.

The restriction enzyme codes used in these figures are:
B: BamHI
C: HincII
E: EcoRI
G: GblII
H: HindII
K: KpnI
M: SmaI
P: PstI
S: SphI
T: SstI
V: PvuII

### 1. Development of TEV-Magainin 2 fusion in T7 Expression Vector.

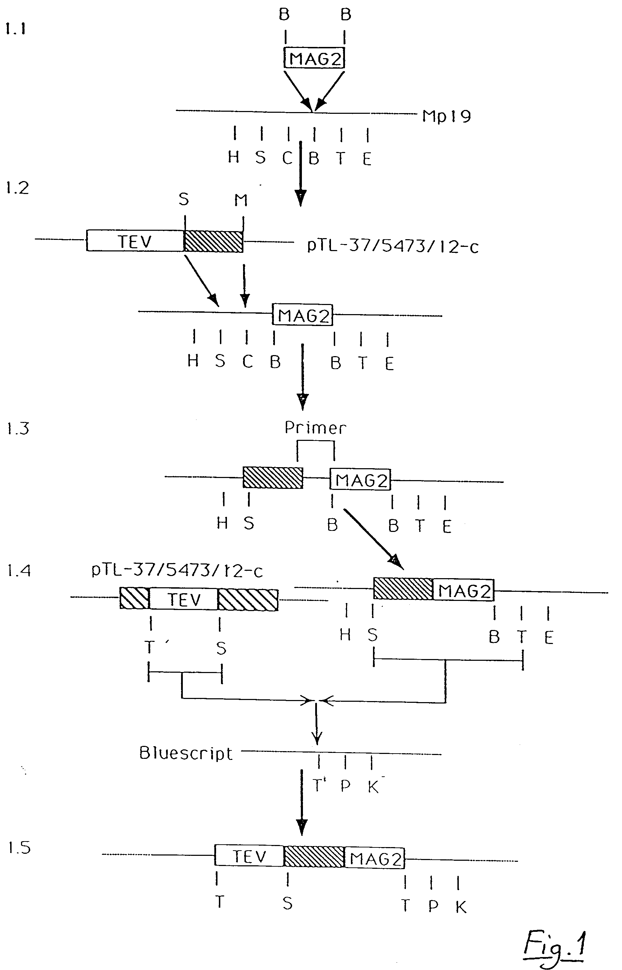

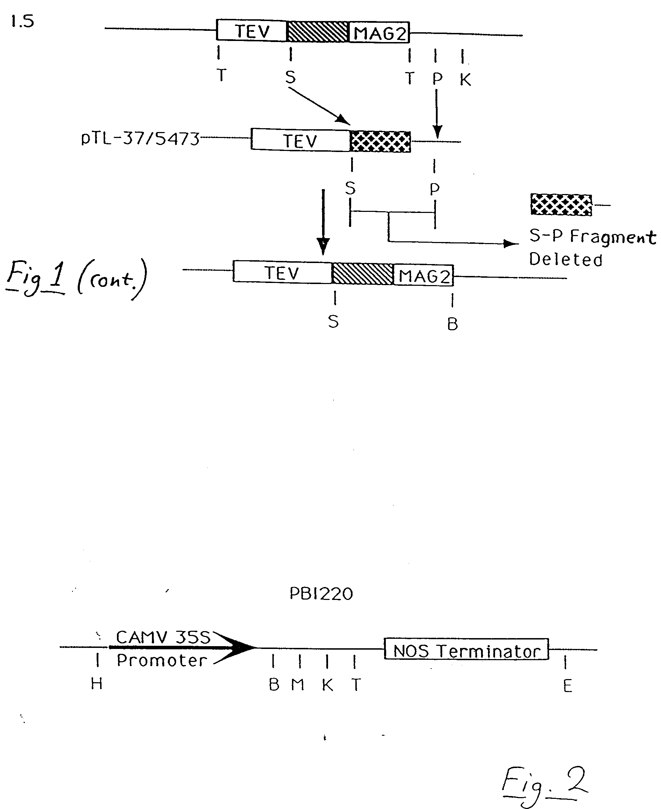

1.1 Two oligonucleotides corresponding to the Magainin 2 coding sequence: 
5'GATCCGGAATTGGTAAATTTTTGCACTCAGCAAAAAAATTTGGAAAAGCT

TTTGTGGGAGAGATAATGAATTCATAAG3'
 and 
5'GATCCTTATGAATTCATTATCTCTCCCACAAAAGCTTTTCCAAATTTTTT
TGCTGAGTGCAAAAATTTACCAATTCCG3')
 were annealed and subcloned into the BamHI site of commercially-available plasmid Mp19. The correct sequence and orientation were verified.
1.2 A SphI/SmaI 465 bp fragment from pTL-37/5473/12-C (Carrington et al, 1987a, 1987b, 1988; Dougherty et al, 1989) was subcloned into the SphI/HincII sites of Mp19 containing Magainin 2.
1.3 Single stranded DNA was isolated and used for site directed mutagenesis using the primer (5'TGCAAAAATTTACCAATTCCTTGCGAGTACACCAATTCATTCAT3') and T4 DNA polymerase. This mutagenesis put the Magainin 2 sequence in frame with the coding region of TEV and reconstructed a proteolytic cleavage site that would allow for the release of Magainin 2. In addition, it removed the remaining polylinker of Mp19 between the Magainin 2 and TEV inserts. The correct sequence was verified.
1.4 A SstI/SphI 1116 bp fragment from pTL-37/5473/12-C and the SstI/SphI fragment from Section 1.3 were ligated together and then subcloned into the SstI site of the "Bluescript" (Stratagene) vector. The orientation and construct configuration were confirmed by restriction analysis.
1.5 The vector pTL-37/5473 and the construct from Section 1.4 were digested with SphI/PstI. The SphI/PstI TEV-Magainin 2 fragment was then inserted into the digested pTL-37/5473 vector. These steps are illustrated in Figure 1.
1.6 Section 1.5 was repeated with the vector pTL-37/5473/His234-Tyr to make a TEV(-)-Magainin 2 construct that had no protease activity due to a substitution of Tyr for His at AA position 234 of the 49kD protease of TEV (Dougherty et al, 1989).

### 2. Construction of TEV-Magainin Plant Expression Vector.

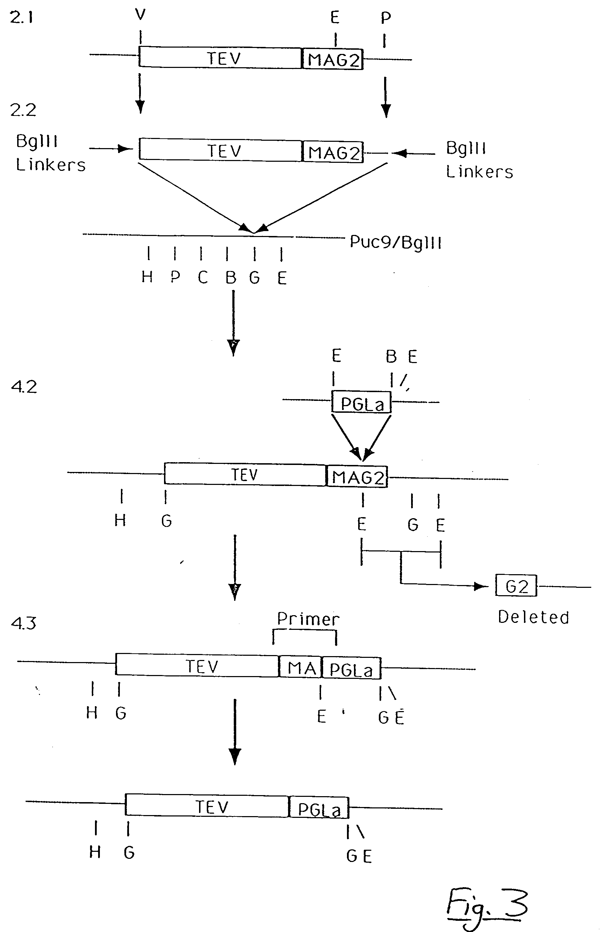

2.1 The TEV (+/-)-Magainin 2 fusions (Sections 1.4 and 1.5) were digested with PvuII and PstI releasing a fragment of about 2266 bp. The DNA was treated with a mixture of T4 DNA polymerase and dNTPs to make blunt ended DNA. BglII linkers (CAGATCTG) were then added to the ends.
2.2 The BglII fragments were then individually cloned into the BglII site of a modified pUC9 vector (BglII linker inserted into the SmaI site). The resulting vector has been deposited as TEV-MAG2 (see below).
2.3 The BglII fragments were then separately subcloned into the BamHI site of the expression cassette Bin19-PBI220 (Figure 2). The correct orientation was determined by restriction analysis.
2.4 The constructs were then transformed into the Agrobacterium strain LBA 4404 using direct DNA transformation. Correct transformants were identified by restriction analysis. All agrobacteria strains were grown on yeast extract (YEP) with appropriate antibiotics. The gene constructs in the Bin 19 vector were introduced into agrobacteria strain LBA4404 by electroporation, and transformants selected on kanamycin.

### 3. Construction of TEV-Magainin F Plant Expression Vector.

3.1 The BglII TEV(-)-Magainin fragment from Section 2.2 was subcloned into the BglII site of a modified pUC119 phagemid (the polylinker from the modified pUC9 vector from Section 2.2 was used to replace the pUC119 polylinker).
3.2 Single-stranded phagemid DNA was isolated and used for site directed mutagenesis using the primer 5'CCGAATTCATTATCTCTGCCACAAAAGCTTTTGCAAATTTTTTTGCTG3' and T7 DNA polymerase. This mutagenesis changed two base pairs that resulted in the coding region of Magainin2 being converted to MagaininF (GIGKFLHSAKKFGKAFVGEIMNSGIGKFLHSAKKFAKAFVAEIMNS).
3.3 A PstI/SpeI fragment from the TEV(-)-Magainin F construct was substituted by the PstI/SpeI fragment from TEV(+)-Magainin 2 to make a TEV(+)-Magainin F construct. This has been deposited as TEV-MAGF (see below).
3.4 The TEV(+/-)-Magainin F constructs were separately put into the expression vector Bin19-PBI220 and the Agrobacterium strain LBA 4404 as in Sections 2.3 and 2.4.

### 4. Construction of TEV-PGLa Plant Expression Vector.

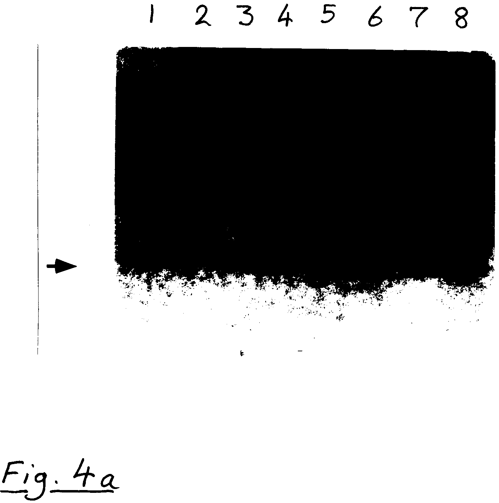

4.1 Two oligonucleotides corresponding to the PGLa peptide sequence: 
(5'AATTCCCGGTACCCCAGATCTATCAAAGAGCCTTAAGAGCAACCTTAGC
AATCTTTCCAGCAATAGCTCCAGCCTTAGAAGCCATTCCCCG3'
 and 
5'AATTCGGGGAATGGCTTCTAAGGCTGGAGCTATTGCTGGAAAGATTGCTA
AGGTTGCTCTTAAGGCTCTTTGATAGATCTGGGGTACCGGG3')
 were annealed and subcloned into the EcoRI site of pUC19 (Figure 3).
4.2 The EcoRI fragment was then inserted into the pUC119 TEV(+)-Magainin 2 construct from Section 2.2, which contained an EcoRI site in the Magainin 2 sequence.
4.3 Single-stranded phagemid DNA was isolated and used for site directed mutagenesis using the primer 5'CCAGCCTTAGAAGCCATTCCTTGCGAGTACACCAATTCATTC3' and T7 DNA polymerase. This resulted in the deletion of the intervening Magainin 2 sequence and put the PGLa sequence in frame with the coding region of TEV. This has been deposited as TEV-PGLa (see below).
4.4 A PstI/SpeI fragment from the TEV(+)-PGLa construct was substituted by the PstI/SpeI fragment from TEV(+)-Magainin 2 to make a TEV(-)-PGLa construct.
4.5 The TEV(+/-)-PGLa constructs were separately put into the expression vector Bin19-PBI220 and the Agrobacterium strain LBA 4404 as in Sections 2.3 and 2.4.

### 5. In-vitro transcription and translation of TEV (+/-)-Magainin 2 construct.

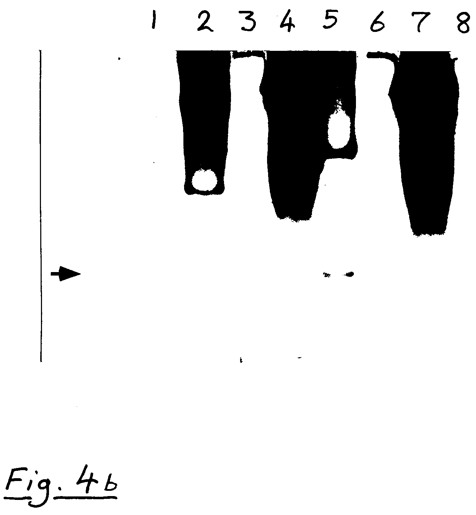

5.1 Transcripts of the TEV (+/-)-Magainin 2 constructs were made using "mCAP RNA Capping Kit" from Stratagene.
5.2 The transcripts were translated using rabbit reticulocyte lysate from Amersham. Each reaction contained 500 ng of mRNA, 120µl of lysate, and 23µl [35S] methionine. The mixture was placed in a 30°C water bath for 2 hours.
5.3 99µl of NET buffer (Sambrook et al, 1989) was added to the reaction mixture.
5.4 A 100µl aliquot was mixed with 10µl of anti-Magainin rabbit antibodies and another aliquot was mixed with 10µl of pre-immune rabbit antibodies. The immunoprecipitation was conducted as described by Sambrook et al (1989) using two washes with NET buffer and one wash with the NP-40 buffer.
5.5 The protein A-Sepharose antibody complex was resuspended in 100µl NET buffer. A 10µl aliquot was removed and centrifuged. The pellet was resuspended in 10µl glacial acetic acid and 10µl 100 mM glycine, pH 1.8.
5.6 The 20µl sample was loaded on a 15% acrylamide-acid gel. The gel was strained in a solution of 0.1% coomassie blue and 50% methanol for one hour. The gel was destained for one hour in 10% acetic acid. The gel was treated with the fluorgraphic reagent "Amplify" from Amersham for 15 minutes. The gel was exposed on X-ray film for 36 hours, and is illustrated in Figure 4.

### Legend for Figures 4a and 4b

Figure 4a depicts an acid-PAGE gel of immunoprecipitated products derived from the in-vitro transcription/translation (labelled with ³⁵S) of the TEV(+/-)-Magainin 2 constructs. All of the samples were spiked, aafter the immunoprecipitation, with 10 µg non-radioactive Magainin 2.

Figure 4b depicts the autoradiograph of this gel. The material in each lane of the gel (identical in both Figures) was as follows. The symbols +/- represent active and inactive proteases, respectively.

### LANE

1. Magainin-2
2. TEV(-)-Magainin 2 construct immunoprecipitated with anti-magainin antibodies.
3. TEV(-)-Magainin 2 construct immunoprecipitated with preimmune antibodies.
4. TEV(-)-Magainin 2 construct translated, but not immunoprecipitated.
5. TEV(+)-Magainin 2 construct immunoprecipitated with anti-magainin antibodies.
6. TEV(+)-Magainin 2 construct immunoprecipitated with preimmune antibodies.
7. TEV(+)-Magainin 2 construct translated, but not immunoprecipitated.
8. Acid extract of skin tissue from Xenopus.

In each gel, the position of the magainin is indicated by the arrow.

### TERMS: Magainin 2: GIGKFLHSAKKFGKAFVGEIMNS Magainin F: GIGKFLHSAKKFAKAFVAEIMNS PGLa: GMASKAGAIAIGKIAKVALKAL TEV(+/-): Active and inactive protease respectively pTL-37/5473/12-c: Is the same construct as pTL-37/5473 except for codon CAA (position 4924) which was changed to CCC, thus creating a SmaI restriction site (Carrington et al, 1988).

### Introduction of gene into potato plants

The plants were derived from tuber sprouts of commercially available varieties. The plants were grown in vitro as shoot cultures at 24°C under continuous light (70µE m² sec⁻¹) in 150 x 25mm glass tubes loosely capped with Magenta lids (Magenta Corp, Chicago, IU, USA) and sealed with gas permeable tape (Micropore Surgical, 3M Company, St. Paul, MN, USA). Nodal stem cuttings with one leaf were taken from 5-6 week old plants and planted in MS (Marashige and Skoog) medium containing 1% sucrose solidified with 0.9% agar (Sigma) and supplemented (after autoclaving) with STS which promoted plant growth and produced larger leaves. The STS (Silver thiosulphate) stock solution (6mM or 1.5 mg/ml) was prepared by adding 12mM AgNO₃ to an equal volume of 96mM Na₂S₂O₃ and used at a concentration of 1µg/ml.

Leaf explants were taken from plantlets at 5-6 weeks after subculturing; cut across the base discarding the petiole and the lower 1-2mm of leaf base, the remaining leaf was used in transformation experiments. Explants were floated overnight on liquid M387. The medium (M387) contained MS salts and vitamins supplemented with 10g/l sucrose, 80mg/l NH₄NO₃, 147mg/l CaC1₂, 10mg/l NAA, 10mg/l BAP. The next day the explants were soaked in an overnight culture of A. Tumefaciens for 15 minutes, blotted dry and placed on solidified M379 callus induction media (MS salts and vitamins supplemented with 1g/l sucrose, 4g/l mannitol, 0.0175mg/l IAA, 2.25mg/l BAP and solidified with 0.8% agar). After two days of co-cultivation the explants were placed on plates with 500ug/ml cefotaxime (Claforan, Roussel) and five to seven days later the explants were transferred to M384 (shoot induction medium, MS supplemented with 15g/l sucrose, 2.25mg/l BAP, 5mg/l giberellin GA₃ and 200mg/l cefotaxime and 50µg/ml kanamycin.

Every three to four weeks the explants were transferred to fresh M384 medium with 200µg/ml cefotaxime and 50µg/l kanamycin. Transformed shoots were removed from the explants and grown in vitro as described above before transfer to soil for further growth.

### Test for blackleg resistance in Magainin-transformed potato plants

Tissue-cultured plants were grown in a glasshouse until they were about 25cm high. Each of five replicates was wounded in two places on the lower part of the stem with a scalpel and inoculated using a suspension of 10⁸ cfu of Erwinina Carotovora subsp. atroseptica per ml of sterile distilled water sprayed into the wounds at a pressure of 1-2 bar. The plants were randomised in blocks and incubated at 20°C and high humidity for 2 days in a chamber before returning them to the glasshouse. Symptoms were scored after 7 days using the following scale:
0 = No infection
1 = Slight necrosis
3 = Restricted lesion
5 = Wilting plant
9 = Dead Plant

The three highest scores per clone were analysed and the mean used as an assessment of resistance. Control plant had a score of 5 or greater.

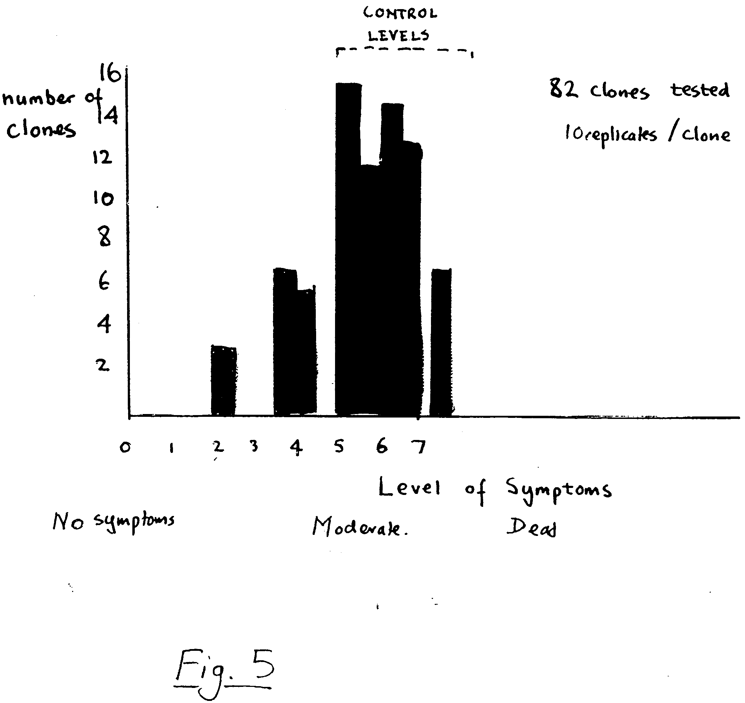

The results (Table 1, Fig. 5) show that some of the transgenic plants had significant resistance to blackleg.

### Deposited plasmids

Three E.coli strains containing plasmids used in the above procedure have been deposited, in accordance with the provisions of the Budapest Treaty, in the National Collections of Industrial and Marine Bacteria, Aberdeen on 5 December 1991 as follows:
- NCTC 40460: : TEV-MAG2
- NCTC 40461: : TEV-MAGF
- NCTC 40462: : TEV-PGLa

**Table 1**

| Transgenic plants inoculated with blackleg | | | |
|---|---|---|---|
| Clone | Mean | Clone | Mean |
| 67l | 2.0 | 74a | 5.7 |
| 61i | 2.3 | 70l | 5.7 |
| 63c | 2.3 | 67t | 5.7 |
| | | 69a | 5.7 |
| 71b | 3.7 | 61f | 5.7 |
| 68g | 3.7 | 71c | 5.7 |
| 208 | 3.7 | 66d | 5.7 |
| 660 | 3.7 | | |
| 68c | 3.7 | X35a | 6.0 |
| 76i | 3.7 | | |
| 71a | 3.7 | 67e | 6.3 |
| | | 71l | 6.3 |
| 69d | 4.3 | 66a | 6.3 |
| 65p | 4.3 | 70h | 6.3 |
| 68e | 4.3 | 62e | 6.3 |
| 67v | 4.3 | 69b | 6.3 |
| 68a | 4.3 | 67z | 6.3 |
| 70i | 4.3 | 70j | 6.3 |
| | | 62a | 6.3 |
| 70s | 5.0 | 71d | 6.3 |
| 71o | 5.0 | 66e | 6.3 |
| 67w | 5.0 | X37 | 6.3 |
| X36b | 5.0 | 61c | 6.3 |
| 71n | 5.0 | 70a | 6.3 |
| 66j | 5.0 | | |
| 67n | 5.0 | 66g | 7.0 |
| 66f | 5.0 | 71e | 7.0 |
| 67i | 5.0 | 69c | 7.0 |
| 71x | 5.0 | 61e | 7.0 |
| 67a | 5.0 | 66b | 7.0 |
| 66z | 5.0 | 67d | 7.0 |
| 65e | 5.0 | 76e | 7.0 |
| 67h | 5.0 | 70d | 7.0 |
| 68h | 5.0 | 65g | 7.0 |
| 65h | 5.0 | 76d | 7.0 |
| | | 70q | 7.0 |
| 72a | 5.7 | 73a | 7.0 |
| 68b | 5.7 | 65a | 7.0 |
| 64a | 5.7 | | |
| 72b | 5.7 | | |
| 68d | 5.7 | | |
| 64b | 7.7 | | |
| 66c | 7.7 | | |
| 70f | 7.7 | | |
| 76h | 7.7 | | |
| 71z | 7.7 | | |
| 76g | 7.7 | | |
| 63b | 7.7 | | |
| 75a | 7.7 | | |
| SED | 1.1 | | |

### References

Bevins and Zasloff (1990): Annu. Rev. Biochem. 59:395-414.
Cannon (1987): Science 328:478.
Carrington et al (1987a): Nucleic Acids Res. 15(23):10066.
Carrington et al (1987b): Virology, 160:355-362.
Carrington et al (1988): Virology, 172:301-310.
Chen et al (1988): FEBS Lett. 236:462-466.
Dougherty et al (1989): Virology 172:302-310.
Giovannini et al (1987): Biochem J. 243:113-120.
Iturriaga et al (1989): The Plant Cell 1:381-390.
Juretic et al (1989): FEBS Lett. 249:219-223.
Marion et al (1988): FEBS Lett. 227:21-26.
Moreira et al (1988): J. Cell Biology 107 (6 part 3):577a.
Poulter et al (1988): J. Biol. Chem. 263:3279-3283. Sambrook et al (1989): Molecular cloning: A laboratory manual. Cold Spring Harbor Laboratory Press 3(18):44-45.
Soravia et al (1988): FEBS Lett. 228:337-340.
Terry et al (1988): J. Biol. Chem. 263:5745-5751.
Urrutia et al (1989): FEBS Lett. 247:17-21.
Westerhoff et al (1989): Biochim. Biophys. Acta 975:361-369.
Westerhoff et al (1989): Proc. Natl. Acad. Sci. USA 86:6597-6601.
Williams et al (1990): Biochemistry, 29:4490-4496.
Zasloff (1987): Proc. Natl. Acad. Sci. USA 84:5449:5453.
Zasloff et al (1988): Proc. Natl. Acad. Sci. USA 85:910-913.

## Claims

1. An expression cassette comprising DNA encoding a peptide comprising a protease recognition site linked directly to the N-terminus of a desired protein.

2. An expression cassette according to claim 1, wherein the desired protein has anti-microbial activity.

3. An expression cassette according to claim 2, wherein the desired protein is a magainin or an analogue thereof, or peptidyl-glycine-leucine carboxyamide (PGLa).

4. An expression vector capable of genetically transforming a host organism, comprising an expression cassette according to any one of claims 1 to 3.

5. A plant transformation expression vector according to claim 4.

6. A Ti plasmid comprising an expression cassette according to any one of claims 1 to 3.

7. A plant cell expressing in biologically active form a magainin or an analogue thereof, or peptidyl-glycine-leucine carboxyamide (PGLa).

8. A higher plant expressing in biologically active form a magainin or an analogue thereof or PGLa.

9. Potato, in the form of whole plants, tubers or a cell culture, having enhanced resistance to bacterial infection conferred by expression therein of a magainin or an analogue thereof, or PGLa.

10. A method of enhancing the resistance of a plant to bacterial infection, comprising the step of genetically transforming the plant by means of a expression vector containing DNA encoding a peptide comprising a protease recognition site linked directly to the N-terminus of a protein having anti-bacterial activity.
